# FASCICULE DE BREVET EUROPEEN

(11) **EP 1 737 837 B1**
(45) Date de publication et mention de la délivrance du brevet: **27.02.2008**
(21) Numéro de dépôt: 05758055.7
(22) Date de dépôt: 19.04.2005
(51) Int. Cl.: C07D 317/50, C07D 317/46, C07D 317/48

(54) **PROCEDE DE PREPARATION DE METHYLENEDIOXYBENZENE EVENTUELLEMENT SUBSTITUE.**
VERFAHREN ZUR HERSTELLUNG VON WAHLWEISE SUBSTITUIERTEM METHYLENDIOXYBENZOL
METHOD FOR PRODUCING OPTIONALLY SUBSTITUTED METHYLENEDIOXYBENZENE

(30) Priorité: 20.04.2004 FR 0404168
(43) Date de publication de la demande: 03.01.2007
(73) Titulaire: RHODIA CHIMIE, 92512 Boulogne Billancourt Cedex (FR)
(72) Inventeur: JACQUOT, Roland, 69340 Francheville (FR); DESMURS, Jean-Roger, 69360 Communay (FR)
(74) Mandataire: Dutruc-Rosset, Marie-Claude
(86) Numéro de dépôt international: PCT/FR2005/000954
(87) Numéro de publication internationale: WO 2005/108385

(56) Documents cités:
- EP-A- 0 261 977
- EP-A- 0 271 091
- LI T-S ET AL: "Montmorillonite clay catalysis. Part 14. A facile synthesis of 2-substituted and 2,2-disubstituted 1,3-benzodioxoles" JOURNAL OF THE CHEMICAL SOCIETY, PERKIN TRANSACTIONS 1, no. 21, 1998, pages 3561-3564, XP002343621

## Description

La présente invention a pour objet un procédé de préparation de méthylènedioxybenzène éventuellement substitué.

Plus précisément, l'invention concerne un procédé de préparation de méthylènedioxybenzène éventuellement substitué, selon un procédé faisant appel à une catalyse hétérogène.

L'invention vise plus particulièrement la préparation de méthylènedioxybenzène.

Dans l'exposé qui suit de la présente invention, on entend par « méthylènedioxybenzène éventuellement substitué », un composé de type méthylènedioxybenzène dont le cycle benzénique est porteur d'un substituant et/ou dont l'un des atomes d'hydrogène du groupe méthylène est substitué par un groupe hydrocarboné.

Le méthylènedioxybenzène est un produit largement utilisé en chimie organique. Il sert notamment comme intermédiaire de synthèse dans le domaine pharmaceutique, agrochimique et en parfumerie.

Plusieurs voies de synthèse sont décrites dans la littérature.

La condensation du pyrocatéchol avec des aldéhydes catalysée par des montmorillonites est décrite dans Journal of the Chemical Society, Perkin Trans. 1, 1998, pages 3561-3564.

Par ailleurs, il est connu de le préparer selon un procédé qui consiste à faire réagir le pyrocatéchol (ou 1,2-dihydroxybenzène) et le chlorure de méthylène, en présence de grandes quantités de soude et d'un agent de transfert de chaîne (FR 2 339 605).

Ce procédé souffre de nombreux inconvénients. Ce procédé est conduit sous pression ce qui implique un équipement spécial.

Il met en oeuvre le chlorure de méthylène qui est un réactif à utiliser avec précautions. L'exposition aux vapeurs peut causer une irritation des voies respiratoires (toux), des yeux, de la peau et des voies digestives provoquant des nausées, vomissements.

Il présente aussi l'inconvénient de s'accompagner de la production de rejets salins comprenant de quantités importantes de chlorure de sodium ce qui implique un surcoût pour traiter les eaux résiduaires.

L'objet de la présente invention est de fournir un procédé permettant d'obvier aux inconvénients précités.

Il a maintenant été trouvé et c'est ce qui fait l'objet de la présente invention, un procédé de préparation de méthylènedioxybenzène éventuellement substitué caractérisé par le fait qu'il comprend la réaction :
- d'un catéchol répondant à la formule (I) : 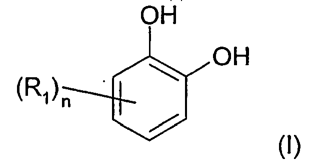 dans ladite formule :
   - R₁ symbolise un substituant sur le noyau benzénique,
   - n, représentant le nombre de substituants, est un nombre allant de 0 à 4, de préférence égal à 0 ou 1,
- et d'un aldéhyde de formule (II) :

   R-CHO (II)

   dans ladite formule, R représente un atome d'hydrogène ou un groupe hydrocarboné ayant de 1 à 12 atomes de carbone,
la réaction ayant lieu en présence d'un catalyseur solide acide choisi parmi : une silicalite de titane et une zéolithe du type zéolithe β dopée par de l'étain et/ou du titane.

Selon un premier mode de réalisation de l'invention, on conduit la réaction en phase vapeur.

Selon un autre mode de réalisation de l'invention, on conduit la réaction en phase liquide.

Conformément au procédé de l'invention, on fait réagir un catéchol éventuellement substitué et un aldéhyde, en présence d'un catalyseur solide tel que défini.

Le procédé de l'invention s'applique particulièrement bien au pyrocatéchol mais convient également aux catéchols substitués représentés par la formule (I).

Comme exemples de catéchols auxquels s'appliquent le procédé de l'invention, on peut citer entre autres ceux qui répondent à la formule (I) dans laquelle R₁ représente un groupe alkyle, un groupe alkoxy ou un atome d'halogène, de préférence un atome de chlore ou de fluor.

Pour ce qui est de l'aldéhyde intervenant dans le procédé de l'invention, il répond particulièrement à la formule (II) dans laquelle R représente un groupe alkyle, cycloalkyle, aryle ou arylalkyle.

Dans le cadre de l'invention, on entend par « alkyle », une chaîne hydrocarbonée linéaire ou ramifiée ayant de 1 à 6 atomes de carbone et de préférence de 1 à 4 atomes de carbone.

Des exemples de groupes alkyle préférés sont notamment méthyle, éthyle, propyle, isopropyle, butyle, isobutyle, t-butyle.

Par « cycloalkyle », on entend un groupe hydrocarboné cyclique, monocyclique comprenant de 3 à 8 atomes de carbone, de préférence, un groupe cyclopentyle ou cyclohexyle.

Par « aryle », on entend un groupe phényle ou un groupe phényle substitué par un ou plusieurs (par exemple 3) substituants. Pour la définition des substituants, on se référera à la signification du groupe R₁.

Par « arylalkyle », on entend un groupe hydrocarboné, linéaire ou ramifié porteur d'un cycle aromatique monocyclique et comprenant de 7 à 12 atomes de carbone, de préférence, benzyle.

Par « alkoxy », on entend un groupe alkyloxy comprenant de 1 à 6 atomes de carbone dans la chaîne alkyle et encore plus préférentiellement de 1 à 4 atomes.

Les catéchols substitués mis en oeuvre préférentiellement dans le procédé de l'invention répondent à la formule (I) dans laquelle R₁ représente un groupe alkyle ayant de 1 à 4 atomes de carbone, de préférence un groupe méthyle ou *tert*-butyle.

Comme exemples préférés de catéchols, on peut citer le pyrocatéchol, le 4-méthylcatéchol, le 4-*tert*-butylcatéchol, le 2,4-*ditert*-butylcatéchol, de préférence le pyrocatéchol.

Pour ce qui est des aldéhydes répondant à la formule (II), on choisit préférentiellement les aldéhydes aliphatiques et notamment le formaldéhyde, l'acétaldéhyde, le propionaldéhyde, le butyraldéhyde, le benzaldéhyde.

On peut mettre en oeuvre l'acétaldéhyde ou toute source d'acétaldéhyde tel que le paraldéhyde.

Le réactif de choix est le formaldéhyde ou tout générateur de formaldéhyde.

On peut faire appel au formaldéhyde ou tout générateur de formaldéhyde tel que, par exemple, le trioxane ou le paraformaldéhyde utilisé sous la forme de polyformaldéhydes linéaires de degré de polymérisation indifférent, ayant de préférence un nombre de motifs (CH₂O) compris entre 8 et 100 motifs,

On peut également mettre en oeuvre l'hexaméthylènetétramine.

On choisit préférentiellement le trioxane.

La quantité d'aldéhyde exprimée en moles d'aldéhyde par mole de catéchol peut varier dans de larges limites. Le rapport molaire catéchol/aldéhyde peut varier entre 1 et 5, et se situe de préférence aux environs de 3.

En ce qui concerne la nature du catalyseur intervenant dans le procédé de l'invention, on fait appel à des catalyseurs de type zéolithique à savoir une silicalite de titane et une zéolithe β dopée.

Dans le présent procédé, intervient une silicalite de titane qui est une zéolithe connue et décrite dans la littérature, en particulier dans " Atlas of zeolites structure types by W. M. Meier and D. H. Olson published by the Structure Commission of the International Zeolite Association (1992), p. 138. "

La composition de la zéolithe peut être représentée par la formule générale :

x TiO₂, (1-x) SiO₂ (I)

dans laquelle x est compris entre 0,0001 et 0,5, de préférence, entre 0,001 et 0,05.

Les silicalites de titane (ou TS-1) qui ont une structure cristalline similaire à la ZSM-5 et qui sont appelées de manière générique « de type pentasile », sont mises en oeuvre préférentiellement.

Leurs propriétés sont décrites dans la littérature : B. Notari, Structure-Activity and Selectivity Relationship in Heterogeneous Catalysis, R.K. Grasselli and A.W. Sleight Editors, Elsevier, 1991, p. 243-256.

La préparation de cette zéolithe peut être conduite selon les procédés décrits notamment dans FR-A-2 471 950 et FR-A-2 489 816 ou suivant le procédé utilisé par M. A. Uguina et al décrit dans J. Chem. Soc. Chem. Commun. P. 27 (1994).

Le procédé selon FR-A-2 471 950 et FR-A-2 489 816 comprend généralement la préparation d'un mélange réactionnel constitué par des sources d'oxyde de silicium et d'oxyde de titane, et éventuellement un oxyde alcalin, d'une base organique azotée et de l'eau ayant une composition dans un rapport molaire de réactifs suivante :
- SiO₂/TiO₂ : 5 à 200, de préférence, 35 à 65,
- OH-/SiO₂ : 0,1 à 1, de préférence, 0,03 à 0,6,
- H₂O/SiO₂ : 20 à 200, de préférence, 60 à 100,
- Me/SiO₂ : 0 à 0,5, de préférence, 0,
- RN⁺/SiO₂ : 0,1 à 2, de préférence, 0,4 à 1,
dans lesdits rapports, Me représente un ion alcalin, de préférence, le sodium ou le potassium, et RN⁺, une base organique azotée.

La source d'oxyde de silicium peut être un orthosilicate de tétraalkyle, de préférence, l'orthosilicate de tétraéthyle ou une silice sous forme colloïdale ou encore un silicate de métal alcalin, de préférence, de sodium ou de potassium.

La source d'oxyde de titane est un composé de titane hydrolysable choisi de préférence, parmi TiCl₄, TiOCl₂ et Ti(alcoxy)₄, de préférence, Ti(OC₂H₅)₄.

La base organique est choisie parmi les hydroxydes d'ammonium quaternaire, préférentiellement, les hydroxydes de tétraalkylammonium, et plus particulièrement de tétrapropylammonium, tétrabutylammonium ou bien parmi les amines telles que notamment la dipropylamine, la tripropylamine, la dibutylamine, la tributylamine.

Le mélange réactionnel est soumis à un traitement hydrothermal, dans un autoclave à une température comprise entre 130°C et 200°C, sous sa propre pression, pendant une durée de 2 à 30 jours, jusqu'à ce que les cristaux du précurseur de zéolithe soient formés.

Ces cristaux sont séparés de la solution mère, soigneusement lavés à l'eau et séchés et chauffés pendant 1 heure à 72 heures, à 550°C, dans de l'air.

La silicalite de titane ainsi obtenue a la composition x TiO₂ (1-x)SiO₂ précisée ci-dessus.

Le procédé utilisé par M. A. Uguina et coll. comprend la préparation d'un cogel séché, amorphe obtenu par hydrolyse contrôlée d'alkoxydes du silicium et du titane (tétraéthylorthosilicate et tétrabutylorthotitanate) puis l'imprégnation de ce cogel par une base organique (hydroxyde de tétrapropylammonium) suivie d'une cristallisation hydrothermale.

Dans un premier temps, on effectue la préparation d'un cogel TiO₂- SiO₂ selon un mode préféré de préparation qui consiste à hydrolyser en milieu acide, une source de silicium puis à ajouter une source de titane. Le sol ainsi préparé est gélifié par ajout d'une base ou par chauffage. On sèche le gel à une température appropriée.

Le rapport molaire TiO₂/SiO₂ dans ce cogel est compris de préférence entre 5 et 200, plus préférentiellement entre 35 et 65.

On hydrolyse la source de silicium dans une solution acide (par exemple, HCl dilué), puis on ajoute la source de titane.

L'opération d'hydrolyse est conduite de préférence à la température ambiante.

On obtient la coprécipitation de TiO₂-SiO₂, par ajout d'une base qui peut être NH₄OH mais l'on préfère faire appel à un hydroxyde de tétralkylammonium qui joue également le rôle d'agent structurant, et plus particulièrement à l'hydroxyde de tétrapropylammonium ou tétrabutylammonium.

Le cogel est séché par exemple une nuit à 110°C.

On réalise l'imprégnation du cogel séché à l'aide d'une solution comprenant l'agent mobilisateur tel que précité puis après l'imprégnation, on fait cristalliser le mélange réactionnel.

La composition du mélange réactionnel est caractérisée par une faible teneur en eau.
- H₂O/SiO₂ : 3 et 10, de préférence aux environs de 4.
- OH-/SiO₂ : 0,1 à 1, de préférence, 0,03 à 0,6,
- RN⁺/SiO₂ : 0,1 à 2, de préférence, 0,2 à 1,
dans ledit rapport RN⁺ représente l'agent structurant organique.

La cristallisation de la zéolithe peut être obtenue par chauffage du cogel pendant le temps nécessaire à la cristallisation, selon le mode opératoire classique de synthèse de zéolithe bien connue de l'Homme du Métier, par exemple 24 heures à 170°C.

En fin de traitement hydrothermal, on sépare le produit obtenu selon les techniques classiques de séparation solide/liquide, de préférence, par filtration.

On le lave, de préférence, à l'eau distillée puis on le soumet à un séchage à une température par exemple de 110°C puis à une calcination par exemple 550°C, pendant 3 heures.

On obtient une silicalite de titane de type MFI qui peut être mise en oeuvre dans le procédé de l'invention.

Elle présente avantageusement une bande d'absorption infra-roùge à 950 - 960 cm-¹.

Il est également possible de faire appel à une silicalite de titane de type MFI et qui est préparé en milieu fluorure. Dans ce cas, la silicalite de titane est appelée "titanozéosilite". Ainsi, on peut notamment faire appel à la titanozéosilite de type MFI qui fait l'objet de WO 00/23185.

La titanozéosilite a un système cristallin orthorhombique et contient du fluor ; la concentration en fluor, après calcination étant avantageusement comprise entre 0,01 et 0,8 %. Les cristaux obtenus se présentent sous la forme de bâtonnets prismatiques de taille moyenne suivante : épaisseur entre 0,1 à 5 µm, de préférence entre 0,2 à 1 µm ; longueur entre 0,5 à 20 µm, de préférence entre 1 et 5 µm ; largeur entre 0,3 et 15 µm, de préférence entre 0,5 et 2,5 µm.

Il est également possible de faire appel à une silicalite de titane de type MEL ou TS-2. Ainsi, on peut notamment faire appel à la silicalite de type MEL qui fait l'objet de WO 00/23377.

La silicalite de titane a un système cristallin quadratique. Les cristaux se présentent sous la forme de parallélépipèdes de taille moyenne suivante : épaisseur entre 50 et 500 nm ; de préférence entre 100 et 250 nm ; longueur entre 100 et 1000 nm, de préférence entre 300 et 500 nm ; largeur entre 50 et 500 nm, de préférence entre 100 et 250 nm.

Un autre type de catalyseur convenant à l'invention et qui est décrit dans WO 03/064363, est un tamis moléculaire micro-poreux de type zéolithe β dans laquelle une partie des atomes de silicium sont substitués par de l'étain et/ou du titane et/ou de l'aluminium.

Le catalyseur intervenant dans le procédé de l'invention, répond à une formule empirique, après calcination qui est la suivante :

(Al_{w} Si_{1-y-z} Sn_{y} Ti_{z})O₂ (II)

dans ladite formule (II) :
- w est un nombre compris entre 0 et 0,2, de préférence entre 0 et 0,1,
- y est un nombre compris entre 0 et 0,1,
- z est un nombre compris entre 0 et 0,1,
- y et z ne peuvent être simultanément égaux à 0.

Les catalyseurs zéolithiques sont des solides micro-poreux dont la taille de ses pores varie généralement entre 5 et 7 Å, et de préférence entre 5,6 et 6,6 Å.

Une première variante du procédé de l'invention consiste à mettre en oeuvre une tamis moléculaire micro-poreux de type zéolithe β à l'étain.

Une autre variante du procédé de l'invention est de faire appel à une tamis moléculaire micro-poreux de type zéolithe β au Ti ou Ti + Al.

Un catalyseur convenant pour la mise en oeuvre du procédé de l'invention est un tamis moléculaire micro-poreux de type zéolithe β à l'étain répondant à une formule empirique, après calcination qui est la suivante :

[Si_{1-y}Sn_{y}]O₂ (IIA)

dans ladite formule (IIA), y est compris entre 0,001 et 0,1, de préférence entre 0,001 et 0,05.

Ledit catalyseur est connu et est décrit notamment dans EP-A 1 010 667.

Un autre type de catalyseur convenant à l'invention est une tamis moléculaire micro-poreux de type zéolithe β au titane répondant à une formule empirique, après calcination qui est la suivante :

[Si_{1-z} Ti_{z}]O₂ (IIB)

dans ladite formule (IIB), z est compris entre 0,001 et 0,1.

Un procédé de préparation d'un tel catalyseur est décrit par T. Blasco et al [J. Phys. Chem. B, 102, 75, 1998].

Il est également possible de faire appel à un catalyseur au titane comprenant du silicium, de l'aluminium qui peut être représenté par la formule empirique, après calcination qui est la suivante :

(Al_{w} Si_{1-z} Ti_{z})O₂ (IIC)

dans ladite formule (IIC) :
- w est un nombre supérieur à 0, compris entre 0 et 0,2, de préférence entre 0,001 et 0,1,
- z est un nombre compris entre 0,001 et 0,1.

Pour la préparation dudit catalyseur, on peut se référer aux travaux de T. Blasco et al, J. Am. Chem. Soc., 115, (1993), p. 11806 - 11813.

Lorsque le catalyseur est de type zéolithique, il peut être mis en oeuvre sous forme de poudre, de granulés, pastilles, billes, pellets ou extrudés, etc...

La zéolithe mise en forme est préférentiellement mise en forme selon la technique d'extrusion.

Parmi les différents catalyseurs précités, la silicalite de titane de type MFI (ou TS-1) est préférée.

Conformément à l'invention, le procédé est conduit en phase gazeuse. On entend par cette expression que les différents réactifs sont vaporisés dans les conditions réactionnelles mais le procédé n'exclut pas la présence d'une éventuelle phase liquide résultant soit des propriétés physiques des réactifs, soit d'une mise en oeuvre sous pression ou de l'utilisation d'un solvant organique.

Le procédé en phase gazeuse est le mode de réalisation préféré de l'invention.

Le gaz vecteur est optionnel et est en général un gaz ou un mélange de gaz non réactifs dans les conditions de la réaction. On peut faire appel à des gaz tels que l'azote, l'air, l'argon ou l'hélium. Avantageusement, le rapport volumique entre le gaz vecteur et les deux réactifs varie entre 0 et 10, de préférence entre 0,1 et 2,0.

La température de la réaction de condensation est généralement comprise entre 200°C et 500°C, et, de préférence entre 250°C et 450°C, et encore plus préférentiellement entre 300°C et 380°C.

La pression réactionnelle est de préférence la pression atmosphérique mais il est également possible de conduire le procédé sous pression réduite pouvant descendre jusqu'à 100 mm de mercure.

Conformément au procédé de l'invention, on vaporise les réactifs de départ à savoir le catéchol et l'aldéhyde, de préférence une source de formaldéhyde. Ils sont introduits au contact du catalyseur, de préférence entraînés par un gaz vecteur.

Une variante de l'invention consiste à utiliser un solvant pour vaporiser les réactifs. Comme exemples de solvants convenant à l'invention, on peut citer en particulier les éthers-oxydes linéaires ou cycliques, de préférence aliphatiques ou cycloaliphatiques notamment le diméthoxyéthane, le dioxane ou le tétrahydrofurane. La quantité mise en oeuvre est définie de telle sorte que les réactifs soient solubilisés à température ambiante.

Le temps de contact qui est défini comme le rapport entre le volume apparent de catalyseur et le débit du flux gazeux (qui inclut le gaz vecteur), peut varier largement, et est le plus souvent compris entre 0,2 et 50 secondes. Le temps de contact est choisi de préférence entre 0,4 et 10 secondes.

En ce qui concerne la réalisation pratique de l'invention, on commence par préparer le lit catalytique qui est constitué par la phase active catalytique déposée sur un support (par exemple, verre fritté ou grille) ce qui permet la circulation des gaz sans élution du catalyseur. Ensuite, les réactifs sont mis en oeuvre et plusieurs variantes sont possibles.

Il est possible de vaporiser chacun des réactifs, dans des chambres différentes, puis d'effectuer le mélange dans une chambre de mélange et d'introduire le flux gazeux résultant sur le catalyseur. Le gaz vecteur peut être introduit en parallèle audit flux gazeux ou bien au niveau de la chambre de mélange.

Une autre variante consiste à préparer une solution comprenant les réactifs, puis à vaporiser ledit mélange et à l'introduire sur le catalyseur, en parallèle avec le gaz vecteur.

Un autre mode d'exécution de l'invention est de faire appel à un solvant organique, inerte dans les conditions réactionnelles et qui est choisi de telle sorte qu'il solubilise les deux réactifs.

Ainsi, on prépare une solution organique comprenant les réactifs, puis l'on vaporise ledit mélange et l'on l'introduit sur le catalyseur, en parallèle avec le gaz vecteur.

En fin de réaction, on condense l'ensemble des gaz et l'on sépare les réactifs non réagis et les produits obtenus, par distillation.

Une autre variante du procédé de l'invention consiste à conduire la réaction en phase liquide.

Dans ce cas, on met en oeuvre un solvant organique.

Le choix du solvant organique est tel qu'il doit satisfaire à certains impératifs.

Il doit être inerte dans les conditions réactionnelles.

Il doit solubiliser les deux réactifs et le produit obtenu à savoir le méthylènedioxybenzène éventuellement substitué.

Comme exemples de solvants, on fait appel aux éthers-oxydes tels que précités.

D'un point de vue pratique, on charge dans un réacteur le catalyseur, de préférence en poudre, le solvant organique, les réactifs et l'on chauffe sous pression autogène des réactifs et solvant.

La température généralement varie entre 100°C et 200°C, de préférence entre 120°C et 150°C.

En fin de réaction, on obtient le méthylènedioxybenzène éventuellement substitué qui peut être représenté par la formule suivante : 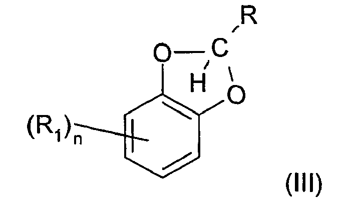 dans ladite formule, R, R₁ et n ont la signification donnée précédemment.

En fin de réaction, on sépare le catalyseur par une technique de séparation solide/liquide, de préférence par filtration.

On récupère le méthylènedioxybenzène éventuellement substitué à partir de la phase liquide de préférence par distillation.

Le présent procédé se prête tout particulièrement bien à une mise en oeuvre continue.

On donne ci-après un exemple de réalisation de l'invention donné à titre illustratif et sans caractère limitatif.

Dans les exemples, on définit le rendement (RR) par le rapport entre le nombre de moles de produit formées et le nombre de moles de substrat engagées.

### Exemple 1

Dans un réacteur phase gaz en verre de diamètre interne 21 mm équipé d'une sonde de température, on introduit successivement 5 ml de poudre de verre, 4 ml de TS-1 (soit 2,2 g) et 5 ml de poudre de verre.

On chauffe le lit catalytique à 330°C sous un courant d'azote de 2 l/h.

On prépare une solution ayant la composition suivante :
- dioxane : 45 g
- pyrocatéchol : 15 g (136 mmol)
- trioxane : 1,36 g (15,1 mmol soit 45,3 mmol de formaldéhyde)

On injecte cette solution à raison de 4 ml/h pendant 5 heures soit 22,8 g de solution injectée.

On récupère ensuite les condensats et l'on dose par chromatographie liquide haute performance le méthylènedioxybenzène formé.

On obtient un rendement de 28 % de méthylènedioxybenzène par rapport aux motifs HCHO injectés.

### Exemple 2

On reproduit l'exemple 1 à la différence près que l'on remplace la TS-1 par de la TS-2.

On obtient un rendement de 30 % de méthylènedioxybenzène par rapport aux motifs HCHO injectés.

### Exemple 3

On reproduit l'exemple 1 à la différence près que l'on remplace le pyrocatéchol par le 4-méthylcatéchol.

On obtient un rendement de 30 % de 4-méthyl-méthylènedioxybenzène par rapport aux motifs HCHO injectés.

### Exemple 4

On met en oeuvre dans cet exemple, le paraldéhyde.

Dans un réacteur en verre de diamètre interne de 21 mm avec sonde de température, on introduit successivement 5 ml de poudre de verre, 4 ml de TS-1 (soit 2,2 g) et 5 ml de poudre de verre.

On chauffe le lit catalytique à 330°C sous un courant de 2 l/h d'azote.

On prépare une solution composée de 45 g de dioxane, 15 g de pyrocatéchol et 2 g de paraldéhyde.

On injecte cette solution à raison de 4 ml/h pendant 5 heures soit 23 g de solution injectée.

On récupère les condensats et l'on dose le produit formé.

On calcule un rendement de 51 % en 2-méthyl-1,3-benzodioxole par rapport aux motifs CH₃CHO engagés.

### Exemple 5

On met en oeuvre dans cet exemple, le propionaldéhyde.

Dans un réacteur en verre de diamètre interne de 21 mm avec sonde de température, on introduit successivement 5 ml de poudre de verre, 4 ml de TS-1 (soit 2,2 g) et 5 ml de poudre de verre.

On chauffe le lit catalytique à 330°C sous un courant de 2 l/h d'azote.

On prépare une solution composée de 45 g de dioxane, 15 g de pyrocatéchol et 2,7 g de propionaldéhyde.

On injecte cette solution à raison de 4 ml/h pendant 5 heures soit 25 g de solution injectée.

On récupère les condensats et l'on dose le produit formé.

On calcule un rendement de 51 % en 2-éthyl-1,3-benzodioxole par rapport au CH₃CH₂CHO engagé.

### Exemple 6

On met en oeuvre dans cet exemple, le benzaldehyde.

Dans un réacteur en verre de diamètre interne de 21 mm avec sonde de température, on introduit successivement 5 ml de poudre de verre, 4 ml de TS-1 (soit 2,2 g) et 5 ml de poudre de verre.

On chauffe le lit catalytique à 330°C sous un courant de 21/h d'azote.

On prépare une solution composée de 45 g de dioxane, 15 g de pyrocatéchol et 4,8 g de benzaldéhyde.

On injecte cette solution à raison de 4 ml /h pendant 5 heures soit 24 g de solution injectée.

On récupère les condensats et l'on dose le produit formé.

On calcule un rendement de 18 % en 2-phényl-1,3-benzodioxole par rapport au benzaldéhyde engagé.

### Exemple 7

On met en oeuvre dans cet exemple, le 4-méthylcatéchol et le trioxane.

Dans un réacteur en verre de diamètre interne de 21 mm avec sonde de température, on introduit successivement 5 ml de poudre de verre, 4 ml de TS-1 (soit 2,2 g) et 5 ml de poudre de verre.

On chauffe le lit catalytique à 330°C sous un courant de 21/h d'azote.

On prépare une solution composée de 45 g de dioxane, 16,9 g de 4-méthylcatéchol et 1,4 g de trioxane.

On injecte cette solution à raison de 4 ml /h pendant 5 heures soit 23 g de solution injectée.

On récupère les condensats et l'on dose le produit formé.

On calcule un rendement de 34 % en 5-méthyl-1,3-benzodioxole par rapport aux motifs HCHO engagés.

## Revendications

1. Procédé de préparation de méthylènedioxybenzène éventuellement substitué **caractérisé par le fait qu'**il comprend la réaction :
- d'un catéchol répondant à la formule (I) : 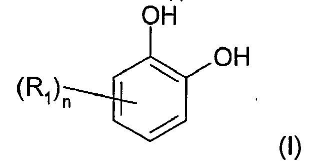 dans ladite formule :
. R₁ symbolise un substituant sur le noyau benzénique,
. n, représentant le nombre de substituants, est un nombre allant de 0 à 4, de préférence égal à 0 ou 1,
- et d'un aldéhyde de formule (II) :
R-CHO (II)
dans ladite formule, R représente un atome d'hydrogène ou un groupe hydrocarboné ayant de 1 à 12 atomes de carbone,
la réaction ayant lieu en présence d'un catalyseur solide acide choisi parmi : une silicalite de titane et une zéolithe du type zéolithe β dopée par de l'étain et/ou du titane.

2. Procédé selon la revendication 1 **caractérisé par le fait que** le catalyseur est une silicalite de titane de type MFI (TS-1) ou de type MEL (TS-2), de préférence une zéolithe TS-1.

3. Procédé selon la revendication 1 **caractérisé par le fait que** le catalyseur est une zéolithe β dopée qui répond après calcination à la formule empirique suivante :
(Al_{w} Si_{1-y-z} Sn_{y} Ti_{z})O₂ (II)
dans ladite formule (II) :
- w est un nombre compris entre 0 et 0,2, de préférence entre 0 et 0,1,
- y est un nombre compris entre 0 et 0,1,
- z est un nombre compris entre 0 et 0,1,
- y et z ne peuvent être simultanément égaux à 0.

4. Procédé selon la revendication 3 **caractérisé par le fait que** le catalyseur est une zéolithe β à l'étain qui répond après calcination, à la formule empirique suivante :
[Si_{1-y}Sn_{y}]O₂ (IIA)
dans ladite formule (IIA), y est compris entre 0,001 et 0,1, de préférence entre 0,001 et 0,05.

5. Procédé selon la revendication 3 **caractérisé par le fait que** le catalyseur est une zéolithe β au titane qui répond après calcination, à la formule empirique suivante :
[Si_{1-z}Ti_{z}]O₂ (IIB)
dans ladite formule (IIB), z est compris entre 0,001 et 0,1.

6. Procédé selon la revendication 3 **caractérisé par le fait que** le catalyseur est une zéolithe β au titane comprenant du silicium, de l'aluminium qui répond après calcination, à la formule empirique suivante :
(Al_{w} Si_{1-z} Ti_{z})O₂ (IIC)
dans ladite formule (IIC) :
- w est un nombre supérieur à 0, compris entre 0 et 0,2, de préférence entre 0,001 et 0,1,
- z est un nombre compris entre 0,001 et 0,1.

7. Procédé selon l'une des revendications 2 à 6 **caractérisé par le fait que** le catalyseur de type zéolithique est mis en oeuvre sous forme de poudre, de granulés, pastilles, billes, pellets ou extrudés.

8. Procédé selon la revendication 1 **caractérisé par le fait que** le catéchol substitué répond à la formule (I) dans laquelle R₁ représente un groupe alkyle, un groupe alkoxy ou un atome d'halogène, de préférence un atome de chlore ou de fluor.

9. Procédé selon la revendication 8 **caractérisé par le fait que** le catéchol substitué répond à la formule (I) dans laquelle R₁ représente un groupe alkyle ayant de 1 à 4 atomes de carbone, de préférence un groupe méthyle ou *tert* butyle.

10. Procédé selon l'une des revendications 8 et 9 **caractérisé par le fait que** le catéchol est le pyrocatéchol, le 4-méthylcatéchol, le 4-*tert*-butylcatéchol, le 2,4-*ditert*-butylcatéchol, de préférence le pyrocatéchol.

11. Procédé selon la revendication 1 **caractérisé par le fait que** l'aldéhyde répond à la formule (II) dans laquelle R représente un atome d'hydrogène, un groupe alkyle, cycloalkyle, aryle ou arylalkyle.

12. Procédé selon la revendication 11 **caractérisé par le fait que** l'aldéhyde est le formaldéhyde, l'acétaldéhyde ou toute source d'acétaldéhyde (paraldéhyde), le propionaldéhyde, le butyraldéhyde, le benzaldéhyde.

13. Procédé selon la revendication 1 **caractérisé par le fait que** l'on met en oeuvre le formaldéhyde ou tout générateur de formaldéhyde de préférence, le trioxane ou le paraformaldéhyde utilisé sous la forme de polyformaldéhydes linéaires de degré de polymérisation indifférent, ayant de préférence un nombre de motifs (CH₂O) compris entre 8 et 100 motifs ou l'hexaméthylènetétramine.

14. Procédé selon la revendication 13 **caractérisé par le fait que** la source de paraformaldéhyde est le trioxane.

15. Procédé selon la revendication 1 **caractérisé par le fait que** le rapport molaire catéchol/aldéhyde varie entre 1 et 5, et se situe de préférence aux environs de 3.

16. Procédé selon l'une des revendications 1 à 10 **caractérisé par le fait que** le procédé est conduit en phase gazeuse.

17. Procédé selon la revendication 16 **caractérisé par le fait que** l'on met en oeuvre un gaz vecteur ou un mélange de gaz non réactifs dans les conditions de la réaction, de préférence l'azote, l'air, l'argon ou l'hélium.

18. Procédé selon la revendication 17 **caractérisé par le fait que** le rapport volumique entre le gaz vecteur et les deux réactifs varie entre 0 et 10, de préférence entre 0,1 et 2,0.

19. Procédé selon la revendication 16 **caractérisé par le fait que** la température de la réaction de condensation est comprise entre 200°C et 500°C, et, de préférence entre 250°C et 450°C, et encore plus préférentiellement entre 300°C et 380°C.

20. Procédé selon la revendication 16 **caractérisé par le fait que** la pression réactionnelle est la pression atmosphérique.

21. Procédé selon la revendication 16 **caractérisé par le fait que** le temps de contact qui est défini comme le rapport entre le volume apparent de catalyseur et le débit du flux gazeux (qui inclut le gaz vecteur) varie entre 0,2 et 50 secondes, et de préférence entre 0,4 et 10 secondes.

22. Procédé selon la revendication 16 **caractérisé par le fait que** l'on met en oeuvre un solvant organique pour vaporiser les réactifs, de préférence un éther-oxyde linéaire ou cyclique, aliphatique ou cycloaliphatique.

23. Procédé selon la revendication 22 **caractérisé par le fait que** le solvant organique est le dioxane.

24. Procédé selon la revendication 16 **caractérisé par le fait que** l'on introduit les réactifs de départ au contact du catalyseur, de préférence entraînés par un gaz vecteur, que l'on condense les gaz en fin de réaction et que l'on sépare le méthylènedioxybenzène éventuellement substitué obtenu.

25. Procédé selon l'une des revendications 1 à 20 **caractérisé par le fait que** le procédé est conduit en phase liquide.

26. Procédé selon la revendication 25 **caractérisé par le fait que** la température varie entre 100°C et 200°C, de préférence entre 120°C et 150°C.

27. Procédé selon la revendication 25 **caractérisé par le fait que** la réaction est conduite dans un solvant organique choisi parmi les éthers-oxydes, linéaires ou cycliques, aliphatiques ou cycloaliphatiques.

28. Procédé selon la revendication 25 **caractérisé par le fait que** l'on charge dans un réacteur le catalyseur, de préférence en poudre, le solvant organique, les réactifs et l'on chauffe sous pression autogène des réactifs et solvant, puis l'on récupère le méthylènedioxybenzène éventuellement substitué obtenu.

## Claims

1. Method for producing optionally substituted methylenedioxybenzene, **characterized in that** it comprises the reaction:
- of a catechol corresponding to formula (I): 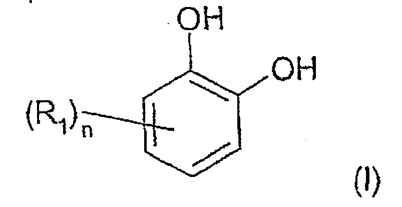 in which said formula:
• R₁ symbolizes a substituent on the benzene ring,
• n, representing the number of substituents, is a number ranging from 0 to 4, preferably equal to 0 or 1,
- and of an aldehyde of formula (II):
R-CHO (II)
in which said formula, R represents a hydrogen atom or a hydrocarbon-based group having from 1 to 12 carbon atoms,
the reaction being carried out in the presence of a solid acid catalyst chosen from: a titanium silicalite and a zeolite of the zeolite β type, that is doped with tin and/or titanium.

2. Method according to Claim 1, **characterized in that** the catalyst is an MFI-type (TS-1) or MEL-type (TS-2) titanium silicalite, preferably a TS-1 zeolite.

3. Method according to Claim 1, **characterized in that** the catalyst is a doped zeolite β, which corresponds, after calcination, to the following empirical formula:
(Al_{w}Si_{1-y-z}Sn_{y}Ti_{z})O₂ (II)
in which said formula (II):
- w is a number between 0 and 0.2, preferably between 0 and 0.1,
- y is a number between 0 and 0.1,
- z is a number between 0 and 0.1,
- y and z cannot simultaneously be equal to 0.

4. Method according to Claim 3, **characterized in that** the catalyst is a tin zeolite β, which corresponds, after calcination, to the following empirical formula:
[Si_{1-y}Sn_{y}]O₂ (IIA)
in which said formula (IIA), y is between 0.001 and 0.1, preferably between 0.001 and 0.05.

5. Method according to Claim 3, **characterized in that** the catalyst is a titanium zeolite β, which corresponds, after calcination, to the following empirical formula:
[Si_{1-z}Ti_{z}]O₂ (IIB)
in which said formula (IIB), z is between 0.001 and 0.1.

6. Method according to Claim 3, **characterized in that** the catalyst is a titanium zeolite β comprising silicon and aluminum, which corresponds, after calcination, to the following empirical formula:
(Al_{w}Si_{1-z}Ti_{z})O₂ (IIC)
in which said formula (IIC):
- w is a number greater than 0, between 0 and 0.2, preferably between 0.001 and 0.1,
- z is a number between 0.001 and 0.1.

7. Method according to one of Claims 2 to 6, **characterized in that** the zeolitic type catalyst is used in the form of a powder, granules, chips, beads, pellets or in extruded form.

8. Method according to Claim 1, **characterized in that** the substituted catechol corresponds to formula (I) in which R₁ represents an alkyl group, an alkoxy group or a halogen atom, preferably a chlorine or fluorine atom.

9. Method according to Claim 8, **characterized in that** the substituted catechol corresponds to formula (I) in which R₁ represents an alkyl group having from 1 to 4 carbon atoms, preferably a methyl or *tert*-butyl group.

10. Method according to either of Claims 8 and 9, **characterized in that** the catechol is pyrocatechol, 4-methylcatechol, 4-*tert*-butylcatechol or 2,4-di-*tert-*butylcatechol, preferably pyrocatechol.

11. Method according to Claim 1, **characterized in that** the aldehyde corresponds to formula (II) in which R represents a hydrogen atom, or an alkyl, cycloalkyl, aryl or arylalkyl group.

12. Method according to Claim 11, **characterized in that** the aldehyde is formaldehyde, acetaldehyde or any source of acetaldehyde (paraldehyde), propionaldehyde, butyraldehyde or benzaldehyde.

13. Method according to Claim 1, **characterized in that** formaldehyde or any formaldehyde-generating agent is used, preferably trioxane or paraformaldehyde, used in the form of linear polyformaldehydes with an indifferent degree of polymerization, preferably having a number of (CH₂O) units of between 8 and 100 units, or hexamethylenetetramine.

14. Method according to Claim 13, **characterized in that** the source of paraformaldehyde is trioxane.

15. Method according to Claim 1, **characterized in that** the catechol/aldehyde molar ratio ranges between 1 and 5, and is preferably in the region of 3.

16. Method according to one of Claims 1 to 10, **characterized in that** the method is carried out in the gas phase.

17. Method according to Claim 16, **characterized in that** a vector gas or a mixture of gases which are not reactive under the reaction conditions, preferably nitrogen, air, argon or helium, is used.

18. Method according to Claim 17, **characterized in that** the ratio by volume of the vector gas to the two reactants ranges between 0 and 10, preferably between 0.1 and 2.0.

19. Method according to Claim 16, **characterized in that** the temperature of the condensation reaction is between 200°C and 500°C, and preferably between 250°C and 450°C, and even more preferably between 300°C and 380°C.

20. Method according to Claim 16, **characterized in that** the reaction pressure is atmospheric pressure.

21. Method according to Claim 16, **characterized in that** the contact time, which is defined as the ratio of the apparent catalyst volume to the flow rate of the gas stream (which includes the vector gas) ranges between 0.2 and 50 seconds, and preferably between 0.4 and 10 seconds.

22. Method according to Claim 16, **characterized in that** an organic solvent is used to vaporize the reactants, preferably a linear or cyclic, aliphatic or cycloaliphatic ether oxide.

23. Method according to Claim 22, **characterized in that** the organic solvent is dioxane.

24. Method according to Claim 16, **characterized in that** the starting reactants are brought into contact with the catalyst, preferably entrained by means of a vector gas, **in that** the gases are condensed at the end of the reaction and **in that** the optionally substituted methylenedioxybenzene obtained is separated.

25. Method according to one of Claims 1 to 20, **characterized in that** the method is carried out in the liquid phase.

26. Method according to Claim 25, **characterized in that** the temperature ranges between 100°C and 200°C, preferably between 120°C and 150°C.

27. Method according to Claim 25, **characterized in that** the reaction is carried out in an organic solvent chosen from linear or cyclic, aliphatic or cycloaliphatic ether oxides.

28. Method according to Claim 25, **characterized in that** the catalyst, preferably in powdered form, the organic solvent and the reactants are loaded into a reactor and heated under autogenic pressure from the reactants and solvent, and then the optionally substituted methylenedioxybenzene obtained is recovered.

## Patentansprüche

1. Verfahren zur Herstellung von Methylendioxybenzol, gegebenenfalls substituiert, **dadurch gekennzeichnet, dass** es die Reaktion umfasst:
- eines Katechols der Formel (I) 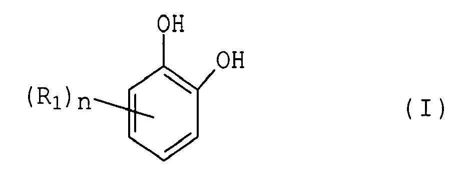 in der
. R₁ einen Substituenten an dem Benzolkern darstellt,
. n die Anzahl der Substituenten darstellt, wobei n eine Zahl ist, die von 0 bis 4 reicht, vorzugsweise gleich 0 oder 1 ist, und
- eines Aldehydes der Formel (II)
R-CHO (II)
in der R ein Wasserstoffatom oder eine KohlenwasserstoffGruppe mit 1 bis 12 Kohlenstoffatomen darstellt,
wobei die Reaktion in Anwesenheit eines festen sauren Katalysators stattfindet, ausgewählt unter: einem Titansilicalit und einem Zeolith vom Typ Zeolith β, dotiert durch Zinn und/oder Titan.

2. Verfahren nach Anspruch 1, **dadurch gekennzeichnet, dass** der Katalysator ein Titansilicalit vom Typ MFI (TS-1) oder vom Typ MEL (TS-2) ist, vorzugsweise ein Zeolith TS-1.

3. Verfahren nach Anspruch 1, **dadurch gekennzeichnet, dass** der Katalysator ein dotierter Zeolith β ist, der nach der Kalzinierung der folgenden empirischen Formel (II) entspricht:
(Al_{w}Si_{1-y-z}Sn_{y}Ti_{z})O₂ (II)
in der
- w eine Zahl zwischen einschließlich 0 und 0,2 ist, vorzugsweise zwischen 0 und 0,1,
- y eine Zahl zwischen einschließlich 0 und 0,1 ist,
- z eine Zahl zwischen einschließlich 0 und 0,1 ist,
- y und z nicht gleichzeitig gleich 0 sein können.

4. Verfahren nach Anspruch 3, **dadurch gekennzeichnet, dass** der Katalysator ein Zeolith β mit Zinn ist, der nach der Kalzinierung der folgenden empirischen Formel (IIA) entspricht:
[Si₁-_{y}Sn_{y}]O₂ (IIA)
in der
y zwischen einschließlich 0,001 und 0,1 beträgt, vorzugsweise zwischen 0,001 und 0,05.

5. Verfahren nach Anspruch 3, **dadurch gekennzeichnet, dass** der Katalysator ein Zeolith β mit Titan ist, der nach der Kalzinierung der folgenden empirischen Formel (IIB) entspricht:
[Si_{1-z}Ti_{z}]O₂ (IIB)
in der
z zwischen einschließlich 0,001 und 0,1 beträgt.

6. Verfahren nach Anspruch 3, **dadurch gekennzeichnet, dass** der Katalysator ein Zeolith β mit Titan ist, der Silicium und Aluminium umfasst und der nach der Kalzinierung der folgenden empirischen Formel (IIC) entspricht:
(Al_{w}Si_{1-z}Ti_{z})O₂ (IIC)
in der
- w eine Zahl von über 0 ist, zwischen einschließlich 0 und 0,2, vorzugsweise zwischen 0,001 und 0,1,
- z eine Zahl zwischen einschließlich 0,001 und 0,1 ist.

7. Verfahren nach einem der Ansprüche 2 bis 6, **dadurch gekennzeichnet, dass** der Katalysator vom Typ Zeolith in Form von Pulver, Granulaten, Pastillen, Kügelchen, Pellets oder Extrudaten eingesetzt wird.

8. Verfahren nach Anspruch 1, **dadurch gekennzeichnet, dass** das substituierte Katechol der Formel (I) entspricht, in der R₁ eine Gruppe Alkyl, eine Gruppe Alkoxy oder ein Halogenatom darstellt, vorzugsweise ein Atom von Chlor oder Fluor.

9. Verfahren nach Anspruch 8, **dadurch gekennzeichnet, dass** das substituierte Katechol der Formel (I) entspricht, in der R₁ eine Gruppe Alkyl mit 1 bis 4 Kohlenstoffatomen darstellt, vorzugsweise eine Gruppe Methyl oder tert.-Butyl.

10. Verfahren nach einem der Ansprüche 8 oder 9, **dadurch gekennzeichnet, dass** das Katechol Pyrokatechol, 4-Methylkatechol, 4-tert.-Butylkatechol, 2,4-ditert-Butylkatechol ist, vorzugsweise Pyrokatechol.

11. Verfahren nach Anspruch 1, **dadurch gekennzeichnet, dass** der Aldehyd der Formel (II) entspricht, in der R ein Wasserstoffatom, eine Gruppe Alkyl, Cycloalkyl, Aryl oder Arylalkyl darstellt.

12. Verfahren nach Anspruch 11, **dadurch gekennzeichnet, dass** der Aldehyd Formaldehyd, Acetaldehyd oder jede Quelle von Acetaldehyd (Paraldehyd), Propionaldehyd, Butyraldehyd, Benzaldehyd ist.

13. Verfahren nach Anspruch 1, **dadurch gekennzeichnet, dass** man den Formaldehyd oder jeden Erzeuger von Formaldehyd, vorzugsweise Trioxan oder Paraformaldehyd, verwendet in Form von linearen Polyformaldehyden mit gleichgültigem Polymerisationsgrad und vorzugsweise einer Anzahl von Struktureinheiten (CH₂O) zwischen einschließlich 8 und 100 Struktureinheiten, oder Hexamethylentetramin einsetzt.

14. Verfahren nach Anspruch 13, **dadurch gekennzeichnet, dass** die Quelle für Paraformaldehyd das Trioxan ist.

15. Verfahren nach Anspruch 1, **dadurch gekennzeichnet, dass** das molare Verhältnis Katechol/Aldehyd zwischen 1 und 5 variiert und vorzugsweise etwa 3 beträgt.

16. Verfahren nach einem der Ansprüche 1 bis 10, **dadurch gekennzeichnet, dass** das Verfahren in der Gasphase durchgeführt wird.

17. Verfahren nach Anspruch 16, **dadurch gekennzeichnet, dass** man ein Trägergas oder eine Mischung von Gasen einsetzt, die unter den Bedingungen der Reaktion nicht reaktiv sind, vorzugsweise Stickstoff, Luft, Argon oder Helium.

18. Verfahren nach Anspruch 17, **dadurch gekennzeichnet, dass** das Volumenverhältnis zwischen dem Trägergas und den zwei Reaktanden zwischen 0 und 10, vorzugsweise zwischen 0,1 und 2,0 variiert.

19. Verfahren nach Anspruch 16, **dadurch gekennzeichnet, dass** die Temperatur der Reaktion der Kondensation zwischen einschließlich 200 °C und 500 °C und vorzugsweise zwischen 250 °C und 450 °C, und noch mehr bevorzugt zwischen 300 °C und 380 °C beträgt.

20. Verfahren nach Anspruch 16, **dadurch gekennzeichnet, dass** der Druck der Reaktion der atmosphärische Druck ist.

21. Verfahren nach Anspruch 16, **dadurch gekennzeichnet, dass** die Zeit des Kontaktes, die als das Verhältnis zwischen dem scheinbaren Volumen des Katalysators und dem Durchsatz des Gasstromes (einschließlich des Trägergases) definiert ist, zwischen 0,2 und 50 Sekunden und vorzugsweise zwischen 0,4 und 10 Sekunden variiert.

22. Verfahren nach Anspruch 16, **dadurch gekennzeichnet, dass** man ein organisches Lösungsmittel zur Verdampfung der Reaktanden einsetzt, vorzugsweise ein lineares oder cyclisches, aliphatisches oder cycloaliphatisches Etheroxid.

23. Verfahren nach Anspruch 23, **dadurch gekennzeichnet, dass** das organische Lösungsmittel Dioxan ist.

24. Verfahren nach Anspruch 16, **dadurch gekennzeichnet, dass** man die Ausgangs-Reaktanden in Kontakt mit dem Katalysator bringt, vorzugsweise mitgeführt durch ein Trägergas, dass man die Gase am Ende der Reaktion kondensiert und dass man das erhaltene, gegebenenfalls substituierte Methylendioxybenzol abtrennt.

25. Verfahren nach einem der Ansprüche 1 bis 20, **dadurch gekennzeichnet, dass** das Verfahren in flüssiger Phase durchgeführt wird.

26. Verfahren nach Anspruch 25, **dadurch gekennzeichnet, dass** die Temperatur zwischen 100 °C und 200 °C, vorzugsweise zwischen 120 °C und 150 °C variiert.

27. Verfahren nach Anspruch 25, **dadurch gekennzeichnet, dass** die Reaktion in einem organischen Lösungsmittel durchgeführt wird, ausgewählt unter den linearen oder cyclischen, aliphatischen oder cycloaliphatischen Etheroxiden.

28. Verfahren nach Anspruch 25, **dadurch gekennzeichnet, dass** man den Katalysator, vorzugsweise in Pulverform, das organische Lösungsmittel und die Reaktanden in einen Reaktor einträgt, dass man Reaktanden und Lösungsmittel unter autogenem Druck erhitzt und dass man anschließend das erhaltene, gegebenenfalls substituierte Methylendioxybenzol gewinnt.
